(19) Europäisches Patentamt
European Patent Office
Office européen des brevets

(11) EP 3 569 265 B1

(12) **EUROPEAN PATENT SPECIFICATION**

(45) Date of publication and mention of the grant of the patent:
**17.03.2021 Bulletin 2021/11**

(21) Application number: **17891180.6**

(22) Date of filing: **28.12.2017**

(51) Int Cl.:
*A61L 29/12* (2006.01) *A61L 17/10* (2006.01)
*A61L 17/14* (2006.01) *A61L 27/16* (2006.01)
*A61L 27/18* (2006.01) *A61L 27/34* (2006.01)
*A61L 27/40* (2006.01) *A61L 27/50* (2006.01)
*A61L 29/04* (2006.01) *A61L 29/06* (2006.01)
*A61L 29/08* (2006.01) *A61L 29/14* (2006.01)
*A61L 31/04* (2006.01) *A61L 31/06* (2006.01)
*A61L 31/10* (2006.01) *A61L 31/12* (2006.01)
*A61L 31/14* (2006.01) *C08F 283/12* (2006.01)
*C08F 290/14* (2006.01) *C08G 77/442* (2006.01)

(86) International application number:
**PCT/JP2017/047349**

(87) International publication number:
**WO 2018/131518 (19.07.2018 Gazette 2018/29)**

(54) **LAMINATE MATERIAL TO BE USED IN LUBRICATIVE MEMBER FOR MEDICAL USE, LUBRICATIVE MEMBER FOR MEDICAL USE, AND MEDICAL DEVICE**

LAMINATMATERIAL ZUR VERWENDUNG IN EINEM SCHMIERELEMENT FÜR MEDIZINISCHE ANWENDUNGEN, SCHMIERELEMENT FÜR MEDIZINISCHE VERWENDUNGEN UND MEDIZINISCHE VORRICHTUNG

MATÉRIAU STRATIFIÉ DESTINÉ À ÊTRE UTILISÉ DANS UN ÉLÉMENT LUBRIFIANT À USAGE MÉDICAL, ÉLÉMENT LUBRIFIANT À USAGE MÉDICAL ET DISPOSITIF MÉDICAL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2017 JP 2017003843**

(43) Date of publication of application:
**20.11.2019 Bulletin 2019/47**

(73) Proprietor: **FUJIFILM Corporation**
**Tokyo 106-8620 (JP)**

(72) Inventors:
- **INOMATA, Sotaro**
**Ashigarakami-gun**
**Kanagawa 258-8577 (JP)**
- **TAKAHASHI, Nobuharu**
**Ashigarakami-gun**
**Kanagawa 258-8577 (JP)**

(74) Representative: **Hoffmann Eitle**
**Patent- und Rechtsanwälte PartmbB**
**Arabellastraße 30**
**81925 München (DE)**

(56) References cited:
**EP-A1- 2 522 375**
**EP-A1- 2 898 905**
**JP-A- 2003 510 134**
**JP-A- 2009 261 437**
**JP-A- 2009 261 437**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 569 265 B1

## Description

## BACKGROUND OF THE INVENTION

1. Field of the Invention

The present invention relates to a medical lubricating member, and a medical device.

2. Description of the Related Art

A medical device to be applied or inserted into the blood vessels, tracheas, gastrointestinal tracts, other body cavities, or tissues in order to examine or treat the human bodies is required not to damage tissues or cause inflammation in the tissues at the time of being brought into contact with the tissues. As such a medical device, for example, JP2009-261437A describes a catheter in which the blood compatibility and the biocompatibility are improved and deterioration of the physical properties and the chemical characteristics due to indwelling in the body for a long period of time is suppressed. This catheter is formed such that at least a part of a body fluid contact portion is coated with an antithrombogenic material formed of a water-insoluble (meth)acrylate copolymer obtained by copolymerizing hydrophobic (meth)acrylate and hydrophilic (meth)acrylate, and a (meth)acrylate copolymer purified to a purity of 95 mol% or greater is used as this (meth)acrylate copolymer.

## SUMMARY OF THE INVENTION

In a case where a medical device is used in contact with the human tissues and the friction between the medical device and the surface of the tissues is large, the tissues are damaged. For example, since an endoscope is used by sliding inside the body cavity, it is important to improve the slipperiness of a surface member of the endoscope to be brought into contact with the tissues inside the body cavity. Since the inside of the body cavity is in a wet state, the surface member of the medical device is required to have improved slipperiness particularly in a wet state.
Further, in a state in which a medical tube is inserted into the body cavity and water is allowed to pass through this tube, the inside of the body cavity is observed or a biopsy is performed by inserting a camera, a jig, or the like thereinto in some cases. In this form, the slipperiness between a jig and the inner wall of the tube in a wet state needs to be improved.
An object of the present invention is to provide a laminated material that enables provision of a medical lubricating member in which the slipperiness is excellent in a wet state and degradation of the slipperiness due to repeated use is unlikely to occur. Further, another object of the present invention is to provide a medical lubricating member in which the slipperiness is excellent in a wet state and degradation of the slipperiness due to repeated use is unlikely to occur. Further, still another object of the present invention is to provide a medical device using the medical lubricating member.
The above-described objects are achieved by the object of the appended claims.

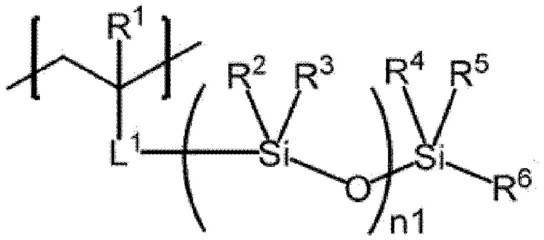

Formula (1)

In the present specification, the numerical value ranges shown using "to" indicate ranges including the numerical values described before and after "to" as the lower limits and the upper limits.
In the present specification, in a case where a plurality of substituents, linking groups, or structural units (hereinafter, referred to as substituents or the like) are denoted by a specific reference or a plurality of substituents or the like are simultaneously or alternatively defined, the plurality of substituents or the like may be the same as or different from one another. The same applies to the definition of the number of substituents or the like. Further, in a case where a plurality of substituents or the like are close to one another (particularly adjacent to one another), this indicates that the substituents or the like may be linked or condensed to form a ring.
In the present specification, the terms "acrylic acid", "acrylamide", and "styrene" are used in a broader sense than usual.
In other words, the concept of "acrylic acid" includes all compounds having a structure of $R^A$-C(=$CR^B_2$)COOH

($R^A$ and $R^B$ each independently represent a hydrogen atom or a substituent).

Further, the concept of "acrylamide" includes all compounds having a structure of $R^C$-C(=C$R^D_2$)CON$R^E_2$ ($R^C$, $R^D$, and $R^E$ each independently represent a hydrogen atom or a substituent).

Further, the concept of "styrene" includes all compounds having a structure of $R^F$-C(=C$R^G_2$)$C_6R^H_6$ ($R^F$, $R^G$, and $R^H$ each independently represent a hydrogen atom or a substituent).

In the present specification, in a case where the number of carbon atoms of a certain group is defined, the number of carbon atoms of this certain group indicates the number of carbon atoms of the whole group. In other words, in a case where this group further has a substituent, the number of carbon atoms thereof indicates the total number of carbon atoms including the number of carbon atoms in this substituent.

In the present specification, the weight-average molecular weight (Mw) and the number average molecular weight (Mn) can be measured as a molecular weight in terms of polystyrene according to GPC unless otherwise specified. At this time, the molecular weight can be measured under a condition of a temperature of 23°C and a flow rate of 1 mL/min using a GPC device HLC-8220 (manufactured by Tosoh Corporation), G3000HXL and G2000HXL as columns, and an RI detector. An eluent can be selected from tetrahydrofuran (THF), chloroform, N-methyl-2-pyrrolidone (NMP), and m-cresol/chloroform (manufactured by Shonan Wako Junyaku K.K.), and THF is used in a case where the target material is dissolved therein.

The molecular weight of a polymer used in a hydrophilic coating layer is measured using N-methyl-2-pyrrolidone (manufactured by Wako Pure Chemical Industries, Ltd.) as an eluent and TSK-gel Super AWM-H (trade name, manufactured by Tosoh Corporation) as a column.

The medical lubricating member or the medical device of the present invention has excellent slipperiness in a wet state and is capable of continuously maintaining the slipperiness. Further, the laminated material used for the medical lubricating member of the present invention enables provision of the medical lubricating member of the present invention.

## BRIEF DESCRIPTION OF THE DRAWINGS

Fig. 1 is a cross-sectional view illustrating an embodiment of a laminated material used for a medical lubricating member of the present invention.

Fig. 2 is a cross-sectional view illustrating an embodiment of a medical lubricating member of the present invention.

## DESCRIPTION OF THE PREFERRED EMBODIMENTS

Preferred embodiments of a laminated material used in a medical lubricating member of the present invention (hereinafter, the "laminated material according to the embodiment of the present invention") will be described.

[Laminated material according to embodiment of present invention]

The laminated material according to the embodiment of the present invention is a material for forming the medical lubricating member according to the embodiment of the present invention described below. The laminated material according to the embodiment of the present invention is a laminate including a base material (hereinafter, also referred to as a "base material a") and a layer (hereinafter, also referred to as a "layer b") which is disposed on the base material a and contains a polymer having a polysiloxane structure described below. The shape of this laminate is not particularly limited, and the surface of the laminated material illustrated in Fig. 1 may be flat or curved. Further, for example, the laminated material is preferably tubular or may be spherical. It is preferable that the layer b is provided directly on the base material a.

<Base material a>

The constituent material of the base material a constituting the laminated material according to the embodiment of the present invention is not particularly limited, and any of materials which can be used for medical devices and the like can be widely applied. For example, glass, plastic, metals, ceramics, fibers, fabrics, paper, leather, synthetic resins, and combinations of these can be used depending on the purpose thereof. Among these, it is preferable that the base material a is formed of a resin. The shape of the base material a is not particularly limited, and the base material a may be plate-like or curved. Further, for example, the base material a is preferably tubular or may be spherical.

The base material a can be suitably used in the present invention even in a case where the surface of the base material a, on which the layer b is formed, has a low surface free energy. For example, the surface free energy of the surface of the base material a, on which the layer b is formed, can be set to be in a range of 5 to 1500 mN/m and also

in a range of 10 to 500 mN/m. Further, the surface free energy of the surface of the base material a, on which the layer b is formed, may be set to be in a range of 5 to 300 mN/m, in a range of 10 to 200 mN/m, in a range of 10 to 100 mN/m, and preferably in a range of 10 to 50 mN/m. In a case where the layer b contains a specific polymer b described below, the layer b can be formed on the base material a without causing cissing or spotting even though the surface free energy of the surface the base material a on which the layer b is formed is low.

The surface free energy can be measured according to a known method. In other words, the surface free energy can be acquired by measuring both of the contact angle of the film with water and the contact angle of film with diiodomethane and substituting the measured values into Owens equation shown below (the following equation is an equation in a case where diiodomethane ($CH_2I_2$) is used as an organic solvent).

Owens Equation

$$1 + \cos\theta_{H2O} = 2(\gamma_s^{d})^{1/2}(\gamma_{H2O}^{d})^{1/2}/\gamma_{H2O,V} + 2(\gamma_s^{h})^{1/2}(\gamma_{H2O}^{h})^{1/2}/\gamma_{H2O,V}$$

$$1 + \cos\theta_{CH2I2} = 2(\gamma_s^{d})^{1/2}(\gamma_{CH2I2}^{d})^{1/2}/\gamma_{CH2I2,V} + 2(\gamma_s^{h})^{1/2}(\gamma_{CH2I2}^{h})^{1/2}/\gamma_{CH2I2,V}$$

Here, $\gamma_{H2O}^{d}$ is 21.8, $\gamma_{H2I2}^{d}$ is 49.5, $\gamma_{H2O}^{h}$ is 51.0, $\gamma_{H2I2}^{h}$ is 1.3, $\gamma_{H2O,V}$ is 72.8, and $\gamma_{CH2I2,V}$ is 50.8, a dispersion force component $\gamma_s^{d}$ of the surface energy and a polar component $\gamma_s^{h}$ are respectively acquired by substituting the measured value of the contact angle of the film with water into $\theta_{H2O}$ and the measured value of the contact angle of the film with diiodomethane into $\theta_{CH2I2}$, and the sum "$\gamma_s^{Vh} = \gamma_s^{d} + \gamma_s^{h}$" can be acquired as the surface free energy (mN/m).

The contact angle is measured by setting the liquid droplet volume of pure water and diiodomethane to 1 μL and reading the contact angle 10 seconds after dropwise addition. At this time, the measurement is performed in an atmosphere of a temperature of 23°C and a relative humidity of 50%.

As the constituent material of the base material a, for example, a urethane resin, a silicon resin, a fluorine resin, an olefin resin, and/or an acrylic resin (at least one of a urethane resin, a silicon resin, a fluorine resin, an olefin resin, or an acrylic resin) can be suitably used. Among these, from the viewpoint of using the material as a medical material, it is more preferable to use a silicon resin.

- Urethane resin -

The urethane resin which can be used as the constituent material of the base material a is not particularly limited. The urethane resin is typically synthesized by performing addition polymerization of a polyisocyanate and a polyol. For example, as the polyisocyanate raw material, aliphatic polyurethane obtained by using aliphatic isocyanate, aromatic polyurethane obtained by using aromatic isocyanate, and a copolymer of these can be used.

Further, as the urethane resin, PANDEX Series (manufactured by DIC Corporation), a urethane resin paint material V-GRAN, V Top Series, and DNT Urethane Smile Clean Series (all manufactured by Dai Nippon Toryo Co., Ltd.), POLYFLEX Series (manufactured by DKS Co., Ltd.), TI-PRENE Series (manufactured by Tiger Polymer Corporation), Tecoflex (registered trademark) Series (manufactured by Thermedics, Inc.), MIRACTORAN Series (manufactured by Nippon Miractoran Co., Ltd.), and PELLETHANE Series (manufactured by The Dow Chemical Company) can be used.

- Silicon resin -

The silicon resin (silicone) which can be used as the constituent material of the base material a is not particularly limited, and a silicon resin formed by being cured using a curing agent may also be used. A typical reaction can be applied as the curing reaction. For example, organohydrogenpolysiloxane and a platinum catalyst can be used, and a peroxide is used in a case of peroxide crosslinking.

Further, as the silicon resin, Rubber Compound KE Series (manufactured by Shin-Etsu chemical Co., Ltd.), ELASTOSIL (registered trademark) Series (manufactured by Wacker Asahikasei Silicone Co., Ltd.), SILASTIC (registered trademark) Series (manufactured by Dow Corning Toray Co., Ltd.), and TSE Series (manufactured by Momentive Performance Materials Inc.) can also be used.

- Fluorine resin -

The fluorine resin which can be used as the constituent material of the base material a is not particularly limited. For example, polytetrafluoroethylene, polyvinyl fluoride, polyvinylidene fluoride, polytrifluoroethylene, and a copolymer thereof can be used.
Further, as the fluorine resin, TEFLON (registered trademark, manufactured by DuPont), POLYFLON and NEOFLON Series (both manufactured by Daikin Industries, Ltd.), Fluon (registered trademark) Series, cytop (registered trademark) series (manufactured by AGC Inc.), and Dyneon Series (manufactured by 3M Company) can also be used.

- Olefin resin -

The olefin resin which can be used as the constituent material of the base material a is not particularly limited. For example, polyethylene, polypropylene, polybutene, polypentene, polycyclopentene, polymethylpentene, polystyrene, polybutadiene, polyisoprene, copolymers of these, or natural rubber can be used. Further, as the olefin resin, ARTON (registered trademark) Series (manufactured by JSR Corporation), SURFLEN (registered trademark) Series (manufactured by Mitsubishi Chemical Corporation), and ZEONOR (registered trademark) Series and ZEONEX (registered trademark) (both manufactured by Zeon Corporation) can also be used.

- Acrylic resin -

The acrylic resin which can be used as the constituent material of the base material a is not particularly limited. For example, an acrylic resin such as methyl polymethacrylate, polymethacrylic acid, methyl polyacrylate, polyacrylic acid, ethyl polymethacrylate, ethyl polyacrylate, or a copolymer of these can be used.
Further, as the acrylic resin, ACRYLITE Series, ACRYPET Series, and ACRYPRENE Series (all manufactured by Mitsubishi Rayon Co., Ltd.), Solvent-based Acrylic Resins for Coatings ACRYDIC Series (manufactured by DIC Corporation), ALMATEX (registered trademark, manufactured by Mitsui Chemicals, Inc.), and HITALOID (manufactured by Hitachi Chemical Company) can also be used.

<Layer b>

In the laminated material according to the embodiment of the present invention, the layer b contains a polymer (hereinafter, also referred to as a "polymer b") having a polysiloxane structure. In a case where the polymer b has a polysiloxane structure, the affinity of the polymer b for the surface of the base material a can be improved even in a case where the surface free energy of the base material a is low, and a layer containing the polymer b can be formed in a state in which cissing or spotting does not occur.
The polymer b contains an acrylic acid component, an acrylic acid ester component, an acrylamide component, and/or a styrene component other than the component having a polysiloxane structure (at least one of an acrylic acid component, an acrylic acid ester component, an acrylamide component, or a styrene component), as the constituent component thereof.
The polymer b contains a hydroxy group, a carboxy group, an amino group, an isocyanate group, an oxazoline ring (oxazolyl group), an epoxy group, a vinyl group, an ethynyl group, a sulfanyl group, an azide group, a trialkoxysilyl group, and/or an acid anhydride structure (at least one of a hydroxy group, a carboxy group, an amino group, an isocyanate group, an oxazoline ring (oxazolyl group), an epoxy group, a vinyl group, an ethynyl group, a sulfanyl group, an azide group, a trialkoxysilyl group, or an acid anhydride structure) (hereinafter, these groups and the structures are also collectively referred to as "reactive functional groups" or simply referred to as "reactive groups"). These reactive functional groups interact with the following hydrophilic polymer which is applied to the layer b or react with this hydrophilic polymer so that the adhesiveness (adhesive force) between the layer b and the hydrophilic polymer is further improved.
It is preferable that the reactive functional group contained in the polymer b is a hydroxy group, a carboxy group, an amino group, an isocyanate group, and/or a trialkoxysilyl group (at least one of a hydroxy group, a carboxy group, an amino group, an isocyanate group, or a trialkoxysilyl group).
It is preferable that the reactive functional group is contained in the acrylic acid component, the acrylic acid ester component, the acrylamide component, and/or the styrene component, which is the constituent component of the polymer b (at least one of the acrylic acid component, the acrylic acid ester component, the acrylamide component, or the styrene component).
As the polymer b, a graft polymer having the polysiloxane structure in a graft chain is preferable. It is preferable that this graft polymer is a structure having a structural unit represented by Formula (1) which has a polysiloxane structure in a graft chain, a structural unit represented by Formula (2) as an acrylic acid component or an acrylic acid ester component, a structural unit represented by Formula (3) as an acrylamide component, and/or a structural unit represented

by Formula (4) as a styrene component (a structure having a structural unit represented by Formula (1) and at least one of a structural unit represented by Formula (2), a structural unit represented by Formula (3), or a structural unit represented by Formula (4)).

- Structural unit having polysiloxane structure in graft chain -

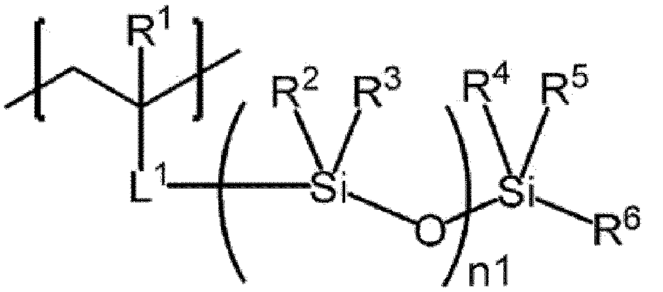

Formula (1)

In Formula (1), $R^1$ to $R^6$ represent a hydrogen atom or an organic group.

Examples of the organic group which can be employed as $R^1$ to $R^6$ include an alkyl group, a cycloalkyl group, an alkenyl group, an aryl group, a heteroaryl group, an alkoxy group, an aryloxy group, a heteroaryloxy group, an alkylthio group, an arylthio group, a heteroarylthio group, an alkylamino group, an arylamino group, a heteroarylamino group, an alkyloxycarbonyl group, an aryloxycarbonyl group, a heteroaryloxycarbonyl group, an alkylaminocarbonyl group, an arylaminocarbonyl group, a heteroarylaminocarbonyl group, and a halogen atom. Among these, an alkyl group, a cycloalkyl group, an alkenyl group, or an aryl group is preferable.

The number of carbon atoms of the alkyl group which can be employed as $R^1$ to $R^6$ is preferably in a range of 1 to 10, more preferably in a range of 1 to 4, still more preferably 1 or 2, and particularly preferably 1. Specific examples of this alkyl group include methyl, ethyl, n-propyl, isopropyl, n-butyl, isobutyl, n-hexyl, n-octyl, 2-ethylhexyl, and n-decyl.

The number of carbon atoms in the cycloalkyl group which can be employed as $R^1$ to $R^6$ is preferably in a range of 3 to 10, more preferably in a range of 5 to 10, and still more preferably 5 or 6. Further, as this cycloalkyl group, a 3-membered ring, a 5-membered ring, or a 6-membered ring is preferable, and a 5-membered ring or a 6-membered ring is more preferable. Specific examples of the cycloalkyl group which can be employed as $R^1$ to $R^6$ include cyclopropyl, cyclopentyl, and cyclohexyl.

The number of carbon atoms of the alkenyl group which can be employed as $R^1$ to $R^6$ is preferably in a range of 2 to 10, more preferably in a range of 2 to 4, and still more preferably 2. Specific examples of the alkenyl group include vinyl, allyl, and butcnyl.

The number of carbon atoms in the aryl group which can be employed as $R^1$ to $R^6$ is preferably in a range of 6 to 12, more preferably in a range of 6 to 10, and still more preferably 6 to 8. Specific examples of this aryl group include phenyl, tolyl, and naphthyl.

As the heteroaryl group which can be employed as $R^1$ to $R^6$, a heteroaryl group having a 5-membered ring or a 6-membered ring which has at least one oxygen atom, sulfur atom, or nitrogen atom is more preferable. Specific examples of this heteroaryl group include 2-pyridyl, 2-thienyl, 2-furanyl, 3-pyridyl, 4-pyridyl, 2-imidazolyl, 2-benzoimidazolyl, 2-thiazolyl, 2-benzothiazolyl, and 2-oxazolyl.

The preferable forms of the aryl group constituting the aryloxy group, the arylthio group, the arylamino group, the aryloxycarbonyl group, and the arylaminocarbonyl group which can be employed as $R^1$ to $R^6$ are the same as the preferable forms of the aryl group which can be employed as $R^1$ to $R^6$.

The preferable forms of the heteroaryl group constituting the heteroaryloxy group, the heteroarylthio group, the heteroarylamino group, the heteroaryloxycarbonyl group, and the heteroarylaminocarbonyl group which can be employed as $R^1$ to $R^6$ are the same as the preferable forms of the heteroaryl group which can be employed as $R^1$ to $R^6$.

The preferable forms of the alkyl group constituting the alkoxy group, the alkylthio group, the alkylamino group, the alkyloxycarbonyl group, and the alkylaminocarbonyl group which can be employed as $R^1$ to $R^6$ are the same as the preferable forms of the alkyl group which can be employed as $R^1$ to $R^6$.

Examples of the halogen atom which can be employed as $R^1$ to $R^6$ include a fluorine atom, a chlorine atom, a bromine atom, and an iodine atom. Among these, a fluorine atom or a bromine atom is preferable.

In a case where $R^1$ to $R^6$ represents an organic group, a form in which the organic group has a substituent may be employed.

$R^1$ to $R^6$ represent preferably an alkyl group, an alkenyl group, or an aryl group and more preferably an alkyl

group having 1 to 4 carbon atoms. Among these, it is preferable that $R^1$ to $R^5$ represent a methyl group, and $R^6$ represents a butyl group.

In Formula (1), $L^1$ represents a single bond or a divalent linking group.

The divalent linking group which can be employed as $L^1$ is not particularly limited as long as the effects of the present invention are exhibited. In a case where $L^1$ represents a divalent linking group, the molecular weight of $L^1$ is preferably in a range of 10 to 200, more preferably in a range of 20 to 100, and still more preferably in a range of 30 to 70.

In a case where $L^1$ represents a divalent linking group, for example, a divalent linking group formed by combining two or more divalent groups selected from an alkylene group, an arylene group, -C(=O)-, -O-, and -$NR^L$- is preferable. $R^L$ represents a hydrogen atom or a substituent. In a case where $R^L$ represents a substituent, an alkyl group is preferable as this substituent. As this alkyl group, an alkyl group having 1 to 6 carbon atoms is preferable, an alkyl group having 1 to 4 carbon atoms is more preferable, and methyl or ethyl is still more preferable.

The alkylene group which can form $L^1$ may be linear or branched. The number of carbon atoms of this alkylene group is preferably in a range of 1 to 10, more preferably in a range of 1 to 6, and still more preferably in a range of 1 to 3.

Further, as the arylene group which can form $L^1$, an arylene group having 6 to 20 carbon atoms is preferable, an arylene group having 6 to 15 carbon atoms is more preferable, an arylene group having 6 to 12 carbon atoms is still more preferable, and a phenylene group is even still more preferable.

It is preferable that $L^1$ represents a divalent linking group formed by combining two or more divalent groups selected from an alkylene group, -C(=O)-, -O-, and -$NR^L$-.

In Formula (1), n1 is an integer of 3 to 10000. In a case where the structural unit represented by Formula (1) has a certain amount of repeating siloxane bonds, the adhesiveness between the base material a and the layer b can be sufficiently improved even in a case where the surface free energy of the surface of the base material a forming the layer b is low. n1 is preferably an integer of 135 to 10000, more preferably an integer of 150 to 5000, and still more preferably an integer of 200 to 1000.

In the polymer b, the content of the structural unit represented by Formula (1) is preferably in a range of 1% to 70% by mass, more preferably in a range of 5% to 60% by mass, and still more preferably in a range of 10% to 50% by mass.

The structural unit represented by Formula (1) can be introduced to the polymer b using a macromonomer having a specific structure as a raw material. This macromonomer can be synthesized according to a method of the related art, or a commercially available product can also be used. Examples of the commercially available product thereof include X-22-174ASX, X-22-174BX, KF-2012, X-22-2426, and X-22-2404 (all trade names, manufactured by Shin-Etsu Chemical co., Ltd.); AK-5, AK-30, and AK-32 (all trade names, manufactured by Toagosei Co., Ltd.); and MCR-M07, MCR-M11, MCR-M17, and MCR-M22 (all trade names, manufactured by Gelest, Inc.).

- Acrylic acid component or acrylic acid ester component -

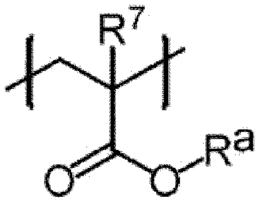

Formula (2)

In Formula (2), $R^7$ and $R^a$ represent a hydrogen atom or an organic group.

Examples of the forms of the organic group which can be employed as $R^7$ include the forms of the organic group which can be employed as $R^1$ in Formula (1). Among these, $R^7$ preferably represents a hydrogen atom or an alkyl group. The number of carbon atoms of the alkyl group is preferably in a range of 1 to 10, more preferably in a range of 1 to 4, still more preferably 1 or 2, and particularly preferably 1. Specific examples of this alkyl group include methyl, ethyl, n-propyl, isopropyl, n-butyl, isobutyl, n-hexyl, n-octyl, 2-ethylhexyl, and n-decyl.

Examples of the forms of the organic group which can be employed as $R^a$ include the forms of the organic group which can be employed as $R^1$ in Formula (1). Among the examples, it is preferable that $R^a$ represents a hydrogen atom, an alkyl group, or an aryl group. The number of carbon atoms of the alkyl group which can be employed as $R^a$ is preferably in a range of 1 to 10 and more preferably in a range of 1 to 6. Specific examples of the alkyl group include methyl, ethyl, n-propyl, isopropyl, n-butyl, isobutyl, n-hexyl, n-octyl, 2-ethylhexyl, and n-decyl.

The number of carbon atoms of the aryl group which can be employed as $R^a$ is preferably in a range of 6 to 12, more preferably in a range of 6 to 10, still more preferably in a range of 6 to 8, and particularly preferably 6. Specific examples of this aryl group include phenyl, tolyl, and naphthyl.

In a case where $R^7$ and $R^a$ represent an organic group, a form in which the organic group has a substituent may be employed. In a case where the polymer b has structural units represented by Formula (2), it is preferable that at least some structural units from among the structural units represented by Formula (2) in the polymer b contain a reactive functional group described above as a substituent.

Further, in the structural units represented by Formula (2) which are present in the polymer b, in a case where $R^a$ represents an alkyl group having a substituent, a form in which $R^a$ is represented by Formula (5) in at least some structural units from among the structural units is also preferable.

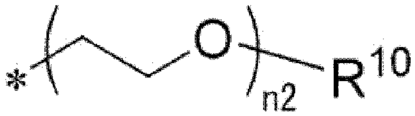
Formula (5)

In Formula (5), n2 is an integer of 1 to 10000. n2 is preferably an integer of 1 to 8000, more preferably an integer of 1 to 5000, and still more preferably an integer of 1 to 3000.

$R^{10}$ represents a hydrogen atom or an organic group. Examples of the forms of the organic group which can be employed as $R^{10}$ include the forms of the organic group which can be employed as $R^1$ in Formula (1). In a case where $R^{10}$ represents an organic group, a form in which the organic group has a substituent may be employed. It is preferable that $R^{10}$ represents a hydrogen atom or an alkyl group. Specific examples of this alkyl group include methyl, ethyl, n-propyl, isopropyl, n-butyl, isobutyl, n-hexyl, n-octyl, 2-ethylhexyl, and n-decyl.

The symbol "*" represents a bonding site with respect to an oxygen atom (-O-) in Formula (2).

In at least some structural units from among the structural units represented by Formula (2) which are present in the polymer b, it is also preferable that $R^a$ represents a nitrogen-containing organic group. The molecular weight of the nitrogen-containing organic group is preferably in a range of 10 to 200 and more preferably in a range of 20 to 100. As the nitrogen-containing organic group, an amino group (including a substituted amino group) is preferable. Preferred examples of the nitrogen-containing organic group include an alkylamino group, an alkylaminoalkyl group, an arylamino group, an arylaminoalkyl group, a heteroarylamino group, and a heteroarylaminoalkyl group.

- Acrylamide component -

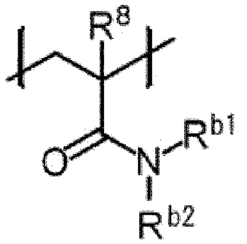
Formula (3)

In Formula (3), $R^8$, $R^{b1}$, and $R^{b2}$ represent a hydrogen atom or an organic group.

Examples of the forms of the organic group which can be employed as $R^8$ include the forms of the organic group which can be employed as $R^1$ in Formula (1). $R^8$ represents preferably a hydrogen atom or an alkyl group and more preferably an alkyl group. The number of carbon atoms of the alkyl group is preferably in a range of 1 to 10, more preferably in a range of 1 to 4, still more preferably 1 or 2, and particularly preferably 1. Specific examples of this alkyl group include methyl, ethyl, n-propyl, isopropyl, n-butyl, isobutyl, n-hexyl, n-octyl, 2-ethylhexyl, and n-decyl.

Examples of the organic groups which can be employed as $R^{b1}$ and $R^{b2}$ include the organic groups which can be employed as $R^1$ in Formula (1). Among the examples, it is preferable that $R^{b1}$ and $R^{b2}$ represent a hydrogen atom, an alkyl group, or an aryl group. The number of carbon atoms of the aryl group is preferably in a range of 6 to 12, more preferably in a range of 6 to 10, still more preferably in a range of 6 to 8, and particularly preferably 6. Specific examples of this aryl group include phenyl, tolyl, and naphthyl.

In a case where $R^8$, $R^{b1}$, and $R^{b2}$ represent an organic group, a form in which the organic group has a substituent may be employed. In a case where the polymer b has structural units represented by Formula (3), it is preferable that at least some structural units from among the structural units represented by Formula (3) in the polymer b contain a reactive functional group described above as a substituent.

- Styrene component -

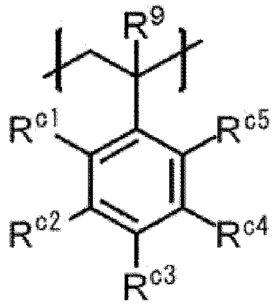

Formula (4)

In Formula (4), $R^9$ represents a hydrogen atom or an organic group. $R^{c1}$ to $R^{c5}$ represent a hydrogen atom, a halogen atom, or an organic group.

Examples of the forms of the organic group which can be employed as $R^9$ include the forms of the organic group which can be employed as $R^1$ in Formula (1). Among these, it is preferable that $R^9$ represents a hydrogen atom.

Examples of the forms of the organic group which can be employed as $R^{c1}$ to $R^{c5}$ include the forms of the organic group which can be employed as $R^1$ in Formula (1). The halogen atom which can be employed as $R^{c1}$ to $R^{c5}$ is not particularly limited. Among examples of the halogen atom, a fluorine atom or a bromine atom is preferable, and a fluorine atom is more preferable. It is preferable that $R^{c1}$ to $R^{c5}$ represent a hydrogen atom, an alkyl group, or a halogen atom. The number of carbon atoms of the alkyl group is preferably in a range of 1 to 10, more preferably in a range of 1 to 4, still more preferably 1 or 2, and particularly preferably 1. Specific examples of this alkyl group include methyl, ethyl, n-propyl, isopropyl, n-butyl, isobutyl, n-hexyl, n-octyl, 2-ethylhexyl, and n-decyl.

In a case where $R^9$ and $R^{c1}$ to $R^{c5}$ represent an organic group, a form in which the organic group has a substituent may be employed. In a case where the polymer b has structural units represented by Formula (4), it is preferable that at least some structural units from among the structural units represented by Formula (4) in the polymer b contain a reactive functional group described above as a substituent.

In a case where the polymer b has a structural unit represented by any of Formulae (2) to (4), the total amount of these structural units in the polymer b is preferably in a range of 10% to 90% by mass, more preferably in a range of 15% to 80% by mass, and still more preferably in a range of 20% to 70% by mass.

Further, in a case where the polymer b is represented by any of Formulae (2) to (4) and has a structural unit that contains the reactive functional group, the content of such a structural unit in the polymer b is preferably in a range of 5% to 70% by mass, more preferably in a range of 10% to 50% by mass, and still more preferably in a range of 15% to 30% by mass.

The polymer b can be synthesized according to a method of the related art. For example, the polymer is obtained by reacting a monomer leading a desired structural unit with a polymerization initiator according to a method of the related art. As the polymerization reaction, any of anionic polymerization, cationic polymerization, and radical polymerization may be used, but radical polymerization is preferable.

The layer b can be formed by preparing a solution (a coating solution for forming the layer b) in which the polymer b is dissolved, coating the base material a with this solution, and drying the solution. Further, this solution may contain a crosslinking agent described below depending on the purpose thereof. Examples the solvent used for the coating solution for forming the layer b include an ether solvent such as dibutyl ether, dimethoxymethane, dimethoxyethane, diethoxyethane, propylene oxide, 1,4-dioxane, 1,3-dioxolane, 1,3,5-trioxane, tetrahydrofuran, anisole, or phenentole; a ketone solvent such as acetone, methyl ethyl ketone, diethyl ketone, dipropyl ketone, diisobutyl ketone, methyl isobutyl ketone, cyclopentanone, cyclohexanone, methyl cyclohexanone, or dimethyl cyclohexanone; an ester solvent such as ethyl formate, propyl formate, n-pentyl formate, methyl acetate, ethyl acetate, methyl propionate, ethyl propionate, n-pentyl acetate, or $\gamma$-butyrolactone; an alcohol solvent such as methanol, ethanol, 1-propanol, 2-propanol, 1-butanol, 2-butanol, tert-butanol, 1-pentanol, 2-methyl-2-butanol, or cyclohexanol; aromatic hydrocarbon such as xylene or toluene; a halogenated hydrocarbon solvent such as methylene chloride, chloroform, or 1,1-dichloroethane; an amide-based solvent such as N-methyl-2-pyrrolidone (NMP), N,N-dimethylformamide (DMF), or N,N-dimethylacetamide (DMAc); a nitrile solvent such as acetonitrile; and an organic solvent containing two or more kinds of functional groups such as methyl 2-methoxy acetate, methyl 2-ethoxy acetate, ethyl 2-ethoxy acetate, ethyl 2-ethoxy propionate, 2-methoxy ethanol, 2-propoxy ethanol, 2-butoxy ethanol, 1,2-diacetoxy acetone, acetyl acetone, diacetone alcohol, methyl acetoacetate, N-methylpyrrolidone, propylene glycol monomethyl ether acetate, or ethyl acetoacetate.

It is also preferable that the polymer b has a crosslinked structure through a crosslinking agent. In this case, it

is preferable that the crosslinking agent is a crosslinking agent (polymeric crosslinking agent) having a structural unit represented by Formula (6) and/or a crosslinking agent represented by formula (7) (at least one of a crosslinking agent having a structural unit represented by Formula (6) or a crosslinking agent represented by Formula (7)). The layer b is cured so that the mechanical strength can be improved by forming the crosslinked structure using these crosslinking agents. These crosslinking agents typically interact with the reactive functional group included in each of the above-described structural units or react with the reactive functional group to form a crosslinked structure in the polymer b. The crosslinking reaction can be carried out according to a method of the related art depending on the kind of the group contributing to the crosslinking reaction.

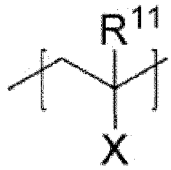 Formula (6)

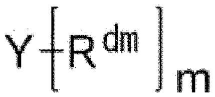 Formula (7)

In Formula (6), $R^{11}$ represents a hydrogen atom or an organic group. In a case where $R^{11}$ represents an organic group, a form in which the organic group has a substituent may be employed. It is preferable that $R^{11}$ represents a hydrogen atom or an alkyl group (an alkyl group having preferably 1 to 5 carbon atoms and more preferably 1 to 3 carbon atoms). X represents a hydroxy group, a carboxy group, an amino group, an isocyanate group, an oxazoline ring, an epoxy group, a vinyl group, an ethynyl group, a sulfanyl group, an azide group, a trialkoxysilyl group, or a group having an acid anhydride structure. X may represent a group having a substituent.

Examples of the crosslinking agent represented by Formula (6) include an oxazoline ring-containing polymer (trade name: EPOCROS (registered trademark), manufactured by Nippon Shokubai Co., Ltd.). The oxazoline ring-containing polymer is a polymer formed of the following structural unit. In the present specification, Me represents methyl.

Further, in a case where the crosslinking agent is a polymer and contains an acrylic acid component, an acrylic acid ester component, an acrylamide component, or a styrene component as a constituent component, these components are respectively included in the acrylic acid component, the acrylic acid ester component, the acrylamide component, or the styrene component defined in the present invention.

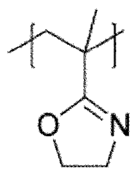 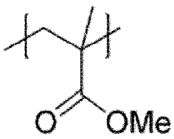 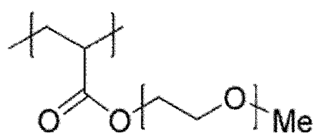

In Formula (7), Y represents an m-valent linking group. It is preferable that Y represents a hydrocarbon group having preferably 2 to 20 carbon atoms and more preferably 2 to 15 carbon atoms. This hydrocarbon group may have heteroatoms in the hydrocarbon chain thereof. Examples of the heteroatoms include O, S, N, and Ti. m is an integer of 2 or greater, preferably an integer or 2 to 8, and more preferably an integer of 2 to 4. $R^{dm}$ has the same definition as that for X in Formula (6).

Examples of the crosslinking agent represented by Formula (7) include a polyisocyanate compound (preferably a diisocyanate compound), a silane coupling agent, and a titanium coupling agent. An example of the crosslinking agent represented by Formula (7) is shown below.

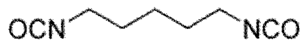

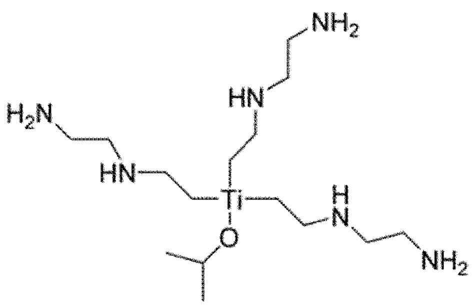

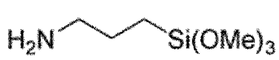

In a case where the polymer b has a crosslinked structure through a crosslinking agent, the proportion of the

crosslinking agent components (the components derived from the crosslinking agent) in the polymer b with the crosslinked structure is preferably in a range of 30% to 90% by mass and more preferably in a range of 40% to 70% by mass.

The weight-average molecular weight of the polymer b (the weight-average molecular weight of the polymer b in a state before being crosslinked in a case where the polymer b has a crosslinked structure through a crosslinking agent) is preferably in a range of 10000 to 300000, more preferably in a range of 30000 to 150000, and still more preferably in a range of 40000 to 120000.

The layer b may be in the form of containing one polymer b or in the form of containing two or more kinds thereof. Further, the content of the polymer b in the layer b is preferably 5% by mass or greater, more preferably 10% by mass or greater, and still more preferably 20% by mass or greater. Further, the content of the polymer b in the layer b is also preferably 40% by mass or greater, preferably 60% by mass or greater, and preferably 80% by mass or greater. In a case where the layer b contains components other than the polymer b, examples of the components other than the polymer b include a polymer binder, a surfactant, polymer fine particles, and inorganic fine particles.

It is preferable that the surface of the layer b is subjected to a hydrophilic treatment. In the present invention, the "surface of the layer b" indicates a surface of the layer b opposite to the surface where the base material a is in contact.

The method of performing a hydrophilic treatment is not particularly limited as long as a hydrophilic group can be imparted to the surface of the layer b (the polymer b which is present in the surface of the layer b). For example, the surface of the layer b can be subjected to a hydrophilic treatment by immersing the layer b in an acidic solution, immersing the layer b in an alkaline solution, immersing the layer b in a peroxide solution, performing a plasma treatment on the layer b, or irradiating the layer b with electron beams.

The thickness of the layer b is typically in a range of 0.01 to 100 μm, preferably in a range of 0.05 to 50 μm, and more preferably in a range of 0.1 to 10 μm.

[Medical lubricating member]

The medical lubricating member according to the embodiment of the present invention is provided by forming a layer c that contains a hydrophilic polymer on the surface of the layer b constituting the laminated material according to the embodiment of the present invention. In other words, the medical lubricating member according to the embodiment of the present invention includes the laminated material according to the embodiment of the present invention and the layer c which is disposed on the layer b (the surface of the layer b) constituting this laminated material and contains a hydrophilic polymer as illustrated in Fig. 2 (Fig. 2 illustrates an example of a medical lubricating member in a case of being produced using the laminated material of Fig. 1). The hydrophilic polymer include polyvinylpyrrolidone, a vinyl ether-maleic acid anhydride copolymer, polyethylene glycol, polyacrylic acid, polyacrylamide, and hyaluronic acid. Further, the hydrophilic polymer can be used alone or in combination of two or more kinds thereof. As the hydrophilic polymer, polyvinylpyrrolidone, vinyl ether-maleic acid anhydride, and/or polyethylene glycol (at least one of polyvinylpyrrolidone, vinyl ether-maleic acid anhydride, or polyethylene glycol) is preferable.

The content of the hydrophilic polymer in the layer c is preferably 50% by mass or greater, more preferably 70% by mass or greater, still more preferably 80% by mass or greater, and particularly preferably 90% by mass or greater. In a case where the layer c contains components other than the hydrophilic polymer, examples of the components other than the hydrophilic polymer include a polymer binder, a surfactant, polymer fine particles, inorganic fine particles, and a crosslinking agent.

The layer c can be formed by preparing a solution (a coating solution for forming the layer c) in which the hydrophilic polymer is dissolved, coating the layer b with this solution, and drying the solution. Further, this solution may contain a crosslinking agent depending on the purpose thereof. Examples the solvent used for the coating solution for forming the layer c include an ether solvent such as dibutyl ether, dimethoxymethane, dimethoxyethane, diethoxyethane, propylene oxide, 1,4-dioxane, 1,3-dioxolane, 1,3,5-trioxane, tetrahydrofuran, anisole, or phenentole; a ketone solvent such as acetone, methyl ethyl ketone, diethyl ketone, dipropyl ketone, diisobutyl ketone, methyl isobutyl ketone, cyclopentanone, cyclohexanone, methyl cyclohcxanonc, or dimethyl cyclohcxanonc; an ester solvent such as ethyl formate, propyl formate, n-pentyl formate, methyl acetate, ethyl acetate, methyl propionate, ethyl propionate, n-pentyl acetate, or γ-butyrolactone; an alcohol solvent such as methanol, ethanol, 1-propanol, 2-propanol, 1-butanol, 2-butanol, tert-butanol, 1-pentanol, 2-methyl-2-butanol, or cyclohexanol; aromatic hydrocarbon such as xylene or toluene; a halogenated hydrocarbon solvent such as methylene chloride, chloroform, or 1,1-dichloroethane; an amide-based solvent such as N-methyl-2-pyrrolidone (NMP), N,N-dimethylformamide (DMF), or N,N-dimethylacetamide (DMAc); a nitrile solvent such as acetonitrile; and an organic solvent containing two or more kinds of functional groups such as methyl 2-methoxy acetate, methyl 2-ethoxy acetate, ethyl 2-ethoxy acetate, ethyl 2-ethoxy propionate, 2-methoxy ethanol, 2-propoxy ethanol, 2-butoxy ethanol, 1,2-diacetoxy acetone, acetyl acetone, diacetone alcohol, methyl acetoacetate, N-methylpyrrolidone, propylene glycol monomethyl ether acetate, or ethyl acetoacetate.

Examples of the crosslinking agent contained in the coating solution for forming the layer c include a polyisocyanate compound (preferably a diisocyanate compound), a silane coupling agent, a titanium coupling agent, a polyepoxy

compound, a polyamine compound, and a melamine compound.

The thickness of the layer c is preferably in a range of 0.1 to 100 μm, more preferably in a range of 0.5 to 50 μm, and still more preferably in a range of 1 to 10 μm.

It is preferable that the medical lubricating member according to the embodiment of the present invention is used as the member of the medical device. The medical lubricating member according to the embodiment of the present invention is typically used such that the layer c forms the outermost surface of the medical device (the inner surface of a tube in a tubular medical device and/or the outer surface of the tube thereof (at least one surface of the inner surface of the tube or the outer surface of the tube)).

The medical device according to the embodiment of the present invention is not particularly limited, and examples thereof include a medical tube, a guide wire, an endoscope, a surgical needle, a surgical suture, forceps, an artificial blood vessel, an artificial heart, and a contact lens. Among these, from the viewpoint of effectively using the slip characteristic of the medical lubricating member according to the embodiment of the present invention in a wet state, an endoscope, a guide wire, a medical tube, or a surgical needle is preferable as the medical device to which the medical lubricating member according to the embodiment of the present invention is applied.

## EXAMPLES

Hereinafter, the present invention will be described in more detail based on the examples. Further, the present invention is not limitatively interpreted by the examples.

<1. Preparation of polymer solution>

[Synthesis Example 1]

16.0 g of a silicone macromer AK-32 (trade name, manufactured by Toagosei Co., Ltd., number average molecular weight of 20000), 4.0 g of hydroxyethyl methacrylate (manufactured by Tokyo Chemical Industry Co., Ltd.), 10.0 g of methoxypolyethylene glycol methacrylate (hereinafter, noted as MPEGMA) (manufactured by Sigma-Aldrich Co. LLC., number average molecular weight of 5000), 10.0 g of methyl methacrylate (manufactured by Tokyo Chemical Industry Co., Ltd.), 0.03 g of azobisisobutyronitrile (AIBN) (manufactured by Wako Pure Chemical Industries, Ltd.), and 60 g of methyl ethyl ketone (MEK) (manufactured by Wako Pure Chemical Industries, Ltd.) were added to a reactor provided with a reflux tower and capable of performing stirring, and the mixture was stirred under conditions of 80°C for 20 hours so as to cause a polymerization reaction. The obtained reaction solution was used as a polymer solution 1. The weight-average molecular weight of the polymer in this polymer solution 1 was 20000.

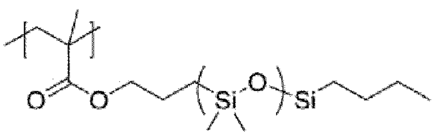
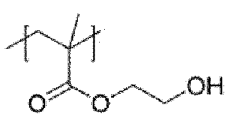
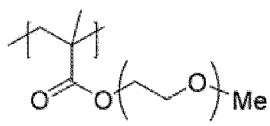
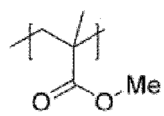

Silicone macromer

Reactive group (OH)

[Synthesis Example 2]

A polymer solution 2 was prepared in the same manner as in Synthesis Example 1 except that 4.0 g of acrylic acid (manufactured by Tokyo Chemical Industry Co., Ltd.) was used in place of 4.0 g of hydroxyethyl methacrylate (manufactured by Tokyo Chemical Industry Co., Ltd.) in Synthesis Example 1. The weight-average molecular weight of the polymer in this polymer solution 2 was 30000.

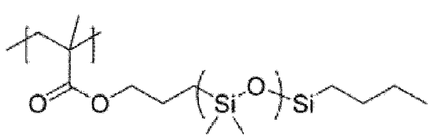
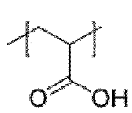
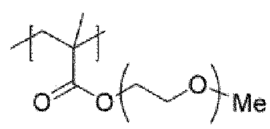
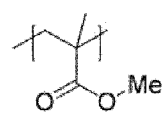

Silicon macromer

Reactive group (CO OH)

[Synthesis Example 3]

A polymer solution 3 was prepared in the same manner as in Synthesis Example 1 except that 4.0 g of 2-(dimethylamino)ethyl methacrylate (manufactured by Tokyo Chemical Industry Co., Ltd.) was used in place of 4.0 g of hydroxyethyl methacrylate (manufactured by Tokyo Chemical Industry Co., Ltd.) in Synthesis Example 1. The weight-average molecular weight of the polymer in this polymer solution 3 was 18000.

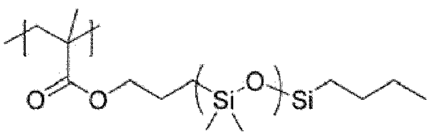
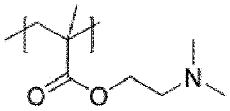
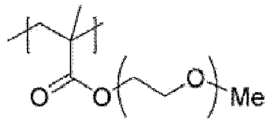
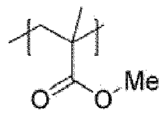

Silicone macromer Reactive group ($NMe_2$)

[Synthesis Example 4]

A polymer solution 4 was prepared in the same manner as in Synthesis Example 1 except that 10.0 g of methyl methacrylate (manufactured by Tokyo Chemical Industry Co., Ltd.) was changed to 20.0 g of methyl methacrylate without using methoxypolyethylene glycol methacrylate (manufactured by Sigma-Aldrich Co. LLC., number average molecular weight of 5000) in Synthesis Example 1. The weight-average molecular weight of the polymer in this polymer solution 4 was 26000.

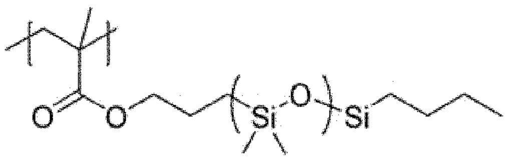
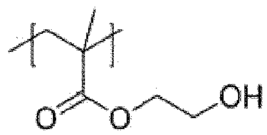
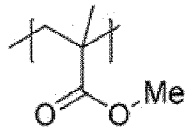

Silicone macromer Reactive group (OH)

[Synthesis Example 5]

A polymer solution 5 was prepared in the same manner as in Synthesis Example 1 except that the silicone macromer of Synthesis Example 1 was changed to X-22-2426 (trade name, manufactured by Shin-Etsu chemical Co., Ltd., number average molecular weight of 12000). The weight-average molecular weight of the polymer in this polymer solution 5 was 30000.

[Synthesis Example 6]

A polymer solution 6 was prepared in the same manner as in Synthesis Example 1 except that the silicone macromer of Synthesis Example 1 was changed to X-24-8201 (trade name, manufactured by Shin-Etsu chemical Co., Ltd., number average molecular weight of 2100). The weight-average molecular weight of the polymer in this polymer solution 6 was 45000.

The charge ratios of raw materials to each polymer solution are collectively listed in Table 1 shown below. In the table, the unit of the blending amount is gram (g), and "-" indicates that the component is not contained.

[Table 1]

| | | Polymer solution | | | | | |
|---|---|---|---|---|---|---|---|
| | | 1 | 2 | 3 | 4 | 5 | 6 |
| Monomer (1) | Silicone macromer AK-32 | 16.0 | 16.0 | 16.0 | 16.0 | - | - |
| | X-22-2426 | - | - | - | - | 16.0 | - |
| | X-24-8201 | - | - | - | - | - | 16.0 |

(continued)

| | | Polymer solution | | | | | |
|---|---|---|---|---|---|---|---|
| | | 1 | 2 | 3 | 4 | 5 | 6 |
| Monomer (2) | Hydroxyethyl methacrylate | 4.0 | - | - | 4.0 | 4.0 | 4.0 |
| | Acrylic acid | - | 4.0 | - | - | - | - |
| | 2-(Dimethylamino)ethyl methacrylate | - | - | 4.0 | - | - | - |
| | MPEGMA | 10.0 | 10.0 | 10.0 | - | 10.0 | 10.0 |
| | Methyl methacrylate | 10.0 | 10.0 | 10.0 | 20.0 | 10.0 | 10.0 |
| Polymerization initiator | Azobisisobutyronitrile | 0.03 | 0.03 | 0.03 | 0.03 | 0.03 | 0.03 |
| Solvent | Methyl ethyl ketone | 60 | 60 | 60 | 60 | 60 | 60 |
| Total | | 100.03 | 100.03 | 100.03 | 100.03 | 100.03 | 100.03 |

<Notes in table>

Silicone macromer AK-32: manufactured by Toagosei Co., Ltd., number average molecular weight of 20000, structure represented by Formula (1)

X-22-2426: manufactured by Shin-Etsu Chemical Co., Ltd., number average molecular weight of 12000, structure represented by Formula (1) (the repetition number of siloxane bonds is different from that of silicone macromer AK-32)
X-24-8201: manufactured by Shin-Etsu Chemical Co., Ltd., number average molecular weight of 2100, structure represented by Formula (1) (the repetition number of siloxane bonds is different from that of silicone macromer AK-32)
MPEGMA: methoxypolyethylene glycol methacrylate, manufactured by Sigma-Aldrich Co. LLC., number average molecular weight of 5000

<2. Preparation of undercoat layer (layer b)>

Each of undercoat liquids 1 to 10 was prepared by dissolving the polymer solution and the crosslinking agent in a solvent at a blending ratio listed in Table 2. In the table, the unit of the blending amount is gram (g), and "-" indicates that the component is not contained.

[Table 2]

| | | Undercoat liquid | | | | | | | | | |
|---|---|---|---|---|---|---|---|---|---|---|---|
| | | 1 | 2 | 3 | 4 | 5 | 6 | 7 | 8 | 9 | 10 |
| Polymer | Polymer solution 1 | 6.3 | - | - | - | - | 6.3 | 12.6 | - | - | - |
| | Polymer solution 2 | - | 6.3 | - | - | - | - | - | - | - | - |
| | Polymer solution 3 | - | - | 6.3 | - | - | - | - | - | - | - |
| | Polymer solution 4 | - | - | - | - | 6.3 | - | - | - | - | - |
| | Polymer solution 5 | - | - | - | - | - | - | - | 6.3 | - | - |
| | Polymer solution 6 | - | - | - | - | - | - | - | - | 6.3 | - |
| | ACRIT 8BS-9000 | - | - | - | 6.3 | - | - | - | - | - | - |
| | Polymer in Synthesis Example 4 of JP2009-261437 A | - | - | - | - | - | - | - | - | - | 2.5 |
| Crosslinking agent | EPOCROS WS-500 | 6.3 | 6.3 | 6.3 | 6.3 | 6.3 | - | - | 6.3 | 6.3 | 6.3 |
| | CORONATE HX | - | - | - | - | - | 2.5 | - | - | - | - |
| Solvent | Isopropanol | 87.4 | 87.4 | 87.4 | 87.4 | 87.4 | 91.2 | 87.4 | 87.4 | 87.4 | 91.2 |
| Total | | 100 | 100 | 100 | 100 | 100 | 100 | 100 | 100 | 100 | 100 |

<Notes in Table>

Polymer solutions 1 to 6: polymer solutions prepared in the above-described manner with solid content of 40% by mass

ACRIT 8BS-9000: thermosetting silicone acrylic polymer, manufactured by Taisei Fine Chemical Co., Ltd., 1.0 mol% of structural unit having polysiloxane bond, solid content of 40% by mass, ACRIT 8BS-9000 has at least the following structural unit

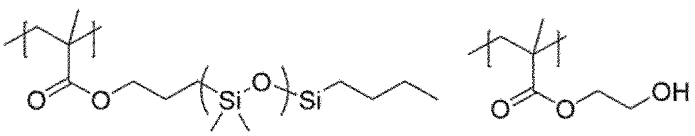

EPOCROS WS-500: oxazoline group-containing polymer, manufactured by Nippon Shokubai Co., Ltd., oxazoline group content of 4.5 mmol/g, solid content of 40% by mass

CORONATE HX: polyisocyanate, manufactured by Tosoh Corporation, isocyanate group content of 20.5% to 22.0% by mass

<3. Preparation of hydrophilic coating solution>

(1) Hydrophilic coating solution 1

A hydrophilic coating solution 1 was prepared by dissolving 2.0 g of polyvinylpyrrolidone (K-90, manufactured by Wako Pure Chemical Industries, Ltd.) and 0.25 g of 4,4-diphenylmethane diisocyanate (MDI) (manufactured by Tokyo Chemical Industry Co., Ltd.) in 100 g of chloroform.

(2) Hydrophilic coating solution 2

A hydrophilic coating solution 2 was prepared by dissolving 2.0 g of a polyvinyl ether-maleic acid anhydride copolymer (number average molecular weight of 311000, manufactured by Sigma-Aldrich Co. LLC.) in 100 g of chloroform.

<4. Preparation of sheet provided with hydrophilic lubricating coating layer (layer c)>

[Example 1]

A silicone rubber sheet (trade name: KE-880-U, hardness of 80A, manufactured by Shin-Etsu Chemical Co., Ltd., surface free energy of 22 mN/m) having a thickness of 500 μm, a width of 50 mm, and a length of 50 mm was immersed in the undercoat liquid 1 for 3 minutes and heated and dried at 150°C for 30 minutes. This dried sheet was immersed in a 10% hydrochloric acid aqueous solution for 12 hours or longer, washed with methanol, and air-dried at room temperature for 1 hour. This air-dried sheet was immersed in the hydrophilic coating solution 1 for 3 minutes and heated and dried at 60°C for 30 minutes and 135°C for 30 minutes to form a hydrophilic lubricating coating layer, thereby preparing a sheet provided with a hydrophilic lubricating coating layer (hereinafter, referred to as a "sheet provided with a hydrophilic coating layer").

[Examples 2 to 9]

Sheets provided with a hydrophilic coating layer of Examples 2 to 9 were prepared in the same manner as in Example 1 except that the undercoat liquids 2 to 9 were used in place of the undercoat liquid 1 of Example 1.

[Example 10]

A sheet provided with a hydrophilic coating layer of Example 10 was prepared in the same manner as in Example 1 except that 10% hydrogen peroxide water was used in place of the 10% hydrochloric acid aqueous solution of Example 1.

[Example 11]

A sheet provided with a hydrophilic coating layer of Example 11 was prepared in the same manner as in Example 1 except that the hydrophilic coating solution 2 was used in place of the hydrophilic coating solution 1 of Example 1.

[Example 12]

A sheet provided with a hydrophilic coating layer of Example 12 was prepared in the same manner as in Example 1 except that a urethane rubber sheet (trade name: 07-007-01, surface free energy of 38 mN/m, manufactured by Hagitec Co., Ltd.) was used in place of the silicone rubber sheet of Example 1.

[Comparative Example 1]

A sheet provided with a hydrophilic coating layer of Comparative Example 1 was prepared in the same manner as in Example 1 except that the undercoat liquid 10 was used in place of the undercoat liquid 1 of Example 1.

<Test>

The following test was performed on each sheet provided with a hydrophilic coating layer prepared in the above-described manner. The test results are collectively listed in Table 3.

[Test Example] Slipperiness and durability

The slipperiness and the durability at the time of wetting were evaluated using a continuous weight type scratch resistance strength tester (type: 18 type (manufactured by HEIDON)). In a state of the sheet prepared in each example was immersed in water, 50 reciprocations of friction were applied to the hydrophilic lubricating coating layer side of the sheet at a load of 500 g using a tetrafluoroethylene indenter, and the dynamic friction coefficients ($\mu k$) at the time of the first reciprocation and the 50th reciprocation were measured.

The "slipperiness" was evaluated using the dynamic friction coefficient at the time of the first reciprocation based on the following evaluation standards. Further, the "durability" was evaluated using the dynamic friction coefficient at the time of the 50th reciprocation based on the following evaluation standards. In both cases, "A" and "B" are in an acceptable level in this test.

<Evaluation standard for slipperiness>

A: $\mu k < 0.03$
B: $0.03 < \mu k < 0.06$
C: $0.06 < \mu k < 0.1$
D: $0.1 < \mu k$

<Evaluation standard for durability>

A: $\mu k < 0.03$
B: $0.03 < \mu k < 0.06$
C: $0.06 < \mu k < 0.1$
D: $0.1 < \mu k$

[Table 3]

| | | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 |
|---|---|---|---|---|---|---|---|---|---|---|---|---|---|---|---|
| Undercoat liquid | | 1 | 2 | 3 | 4 | 5 | 6 | 7 | 8 | 9 | 1 | 1 | 1 | 10 | - |
| Undercoat polymer/(crosslinking agent + undercoat polymer) × 100[*1] | | 50 | 50 | 50 | 50 | 50 | 50 | 100 | 50 | 50 | 50 | 50 | 50 | 50 | - |
| Degree of polymerization of dimethylsiloxane in silicone macromer | | 270 | 270 | 270 | 270 | 270 | 270 | 270 | 161 | 27 | 270 | 270 | 270 | 27 | - |
| Hydrophilic step | | 10% Hydrochloric acid | 10% Hydrochloric acid | 10% Hydrochloric acid | 10% Hydrochloric acid | 10% Hydrochloric acid | 10% Hydrochloric acid | 10% Hydrochloric acid | 10% Hydrochloric acid | 10% Hydrochloric acid | 10% Hydrogen peroxide water | 10% Hydrochloric acid | 10% Hydrochloric acid | 10% Hydrochloric acid | 10% Hydrochloric acid |
| Hydrophilic coating layer | | PVP + MDI | PVP + MDI | PVP + MDI | PVP + MDI | PVP + MDI | PVP + MDI | PVP + MDI | PVP + MDI | PVP + MDI | PVP + MDI | Maleic acid anhydride-vinyl ether copolymer | PVP + MDI | PVP + MDI | PVP + MDI |
| Base material | | Silicone rubber | Silicone rubber | Silicone rubber | Silicone rubber | Silicone rubber | Silicone rubber | Silicone rubber | Silicone rubber | Silicone rubber | Silicone rubber | Silicone rubber | Urethane rubber | Silicone rubber | Silicone rubber |
| Evaluation | Slipperiness | A | A | A | A | A | A | A | A | A | A | A | A | B | D |
| | Durability | A | A | A | A | B | A | B | A | B | A | A | A | D | D |

<Notes in table>

PVP: polyvinylpyrrolidone (weight-average molecular weight of 600000)
MDI: diphenylmethane diisocyanate
Maleic acid anhydride-vinyl ether copolymer: weight-average molecular weight of 1080000, copolymerization ratio of maleic acid anhydride to vinyl ether = 50/50 (molar ratio)
*1: % by mass

As shown in Table 3, in a case where the polymer used for forming the undercoat layer (layer b) did not contain a reactive functional group, the adhesiveness between the undercoat layer and the hydrophilic coating layer was degraded, and the slipperiness was deteriorated due to repeated sliding (Comparative Example 1). Further, in a case where the hydrophilic coating layer was formed without providing the undercoat layer, the slipperiness was deteriorated (Comparative Example 2).

On the contrary, in the forms of Examples 1 to 12 satisfying the definition of the present invention, the slipperiness was excellent and was sufficiently maintained in spite of repeated sliding.

Explanation of References

10: laminated material
20: medical lubricating member
a: base material
b: layer containing polymer having polysiloxane structure
c: layer containing hydrophilic polymer

## Claims

1. A medical lubricating member, comprising a laminated material comprising

    (i) a base material (a);
    (ii) disposed on the base material (a), a layer (b) containing a polymer which has a polysiloxane structure and contains

    - an acrylic acid component, an acrylic acid ester component, an acrylamide component, and/or a styrene component as a constituent component, and
    - a hydroxy group, a carboxy group, an amino group, an isocyanate group, an oxazoline ring, an epoxy group, a vinyl group, an ethynyl group, a sulfanyl group, an azide group, a trialkoxysilyl group, and/or an acid anhydride structure in a molecule; and

    (iii) disposed on the layer (b), a layer (c) containing a hydrophilic polymer selected from one or a combination of two or more of polyvinylpyrrolidone, vinyl ether-maleic acid anhydride copolymer, polyethylene glycol, polyacrylic acid, polyacrylamide and hyaluronic acid.

2. The medical lubricating member of claim 1, wherein the polymer of the layer (b) is a graft polymer having the polysiloxane structure in a graft chain.

3. The medical lubricating member of claim 1 or 2, wherein the polymer of the layer (b) contains a structural unit of formula (1) and a structural unit of formula (2), a structural unit of formula (3), and/or a structural unit of formula (4):

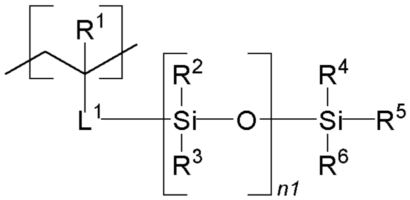 (1)

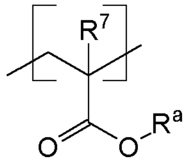 (2)

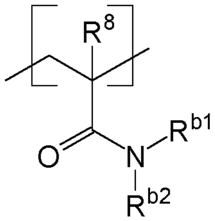 (3)

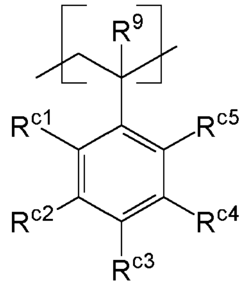 (4)

wherein

$R^1$-$R^9$, $R^a$, $R^{b1}$ and $R^{b2}$ are H or an organic group,
$R^{c1}$-$R^{c5}$ are H, halogen or an organic group
$L^1$ is a single bond or a divalent linking group, and
*n1* is an integer of 3-10,000.

**4.** The medical lubricating member of claim 3, wherein $R^a$ is a group of formula (5) or a nitrogen-containing organic group,

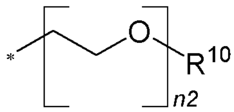 (5)

wherein n2 is an integer of 1-10,000, $R^{10}$ is H or an organic group, and the symbol "*" is a bonding site.

**5.** The medical lubricating member of claim 3 or 4, wherein n1 is 135-10,000.

**6.** The medical lubricating member of any of claims 1-5, wherein the polymer of the layer (b) is a crosslinked body having a crosslinked structure formed of a crosslinking agent component from a structural unit of formula (6) and/or a compound of formula (7):

$-[CH_2-C(R^{11})(X)]-$ (6)

$Y-(R^{dm})_m$ (7)

wherein

$R^{11}$ is H or an organic group,
X, $R^{dm}$ each independently are OH, carboxy, amino, isocyanate, an oxazoline ring, epoxy, vinyl, ethynyl, sulfanyl, azide, trialkoxysilyl or a group having an acid anhydride structure,
Y is an *m*-valent linking group, and
*m* is an integer of ≥ 2.

**7.** The medical lubricating member of claim 6, wherein the proportion of the crosslinking agent component in the crosslinked body of the layer (b) is 30-90 mass-%.

**8.** The medical lubricating member of any of claims 1-7,

wherein a surface of the layer (b) is subjected to a hydrophilic treatment.

9. The medical lubricating member of any of claims 1-8, wherein the base material a is formed of a urethane resin, a silicon resin, a fluorine resin, an olefin resin and/or an acrylic resin.

10. The medical lubricating member of any of claims 1-9, wherein the base material a is a silicon resin.

11. The use of the medical lubricating member of any of claims 1-10 as a member of a medical device selected from a medical tube, a guide wire, an endoscope, a surgical needle, a surgical suture, forceps, an artificial blood vessel, an artificial heart, and a contact lens.

12. A medical device which comprises the medical lubricating member of any of claims 1-10 and is selected from a medical tube, a guide wire, an endoscope, a surgical needle, a surgical suture, forceps, an artificial blood vessel, an artificial heart, and a contact lens.

## Patentansprüche

1. Medizinisches Schmierelement, umfassend ein laminiertes Material, umfassend

(i) ein Basismaterial (a),
(ii) auf dem Basismaterial (a) angeordnet eine Schicht (b), die ein Polymer enthält, das eine Polysiloxanstruktur aufweist und enthält:

- eine Acrylsäurekomponente, eine Acrylsäureesterkomponente, eine Acrylamidkomponente und/oder eine Styrolkomponente als Bestandteil und
- eine Hydroxygruppe, eine Carboxygruppe, eine Aminogruppe, eine Isocyanatgruppe, einen Oxazolinring, eine Epoxygruppe, eine Vinylgruppe, eine Ethinylgruppe, eine Sulfanylgruppe, eine Azidgruppe, eine Trialkoxysilylgruppe und/oder eine Säureanhydridstruktur in einem Molekül, und

(iii) angeordnet auf der Schicht (b) eine Schicht (c), die ein hydrophiles Polymer, ausgewählt aus einem oder einer Kombination aus zwei oder mehr von Polyvinylpyrrolidon, Vinylether-Maleinsäureanhyrid-Copolymer, Polyethylenglykol, Polacrylsäure, Polyacrylamid und Hyaluronsäure, enthält.

2. Medizinisches Schmierelement gemäß Anspruch 1, wobei das Polymer der Schicht (b) ein Pfropfpolymer mit der Polysiloxanstruktur in einer Pfropfkette ist.

3. Medizinisches Schmierelement gemäß Anspruch 1 oder 2, wobei das Polymer der Schicht (b) eine Struktureinheit der Formel (1) und eine Struktureinheit der Formel (2), eine Struktureinheit der Formel (3) und/oder eine Struktureinheit der Formel (4) enthält:

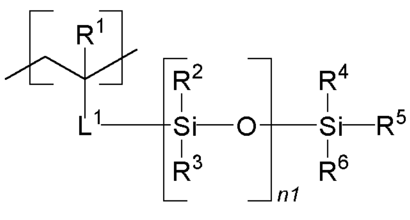
(1)

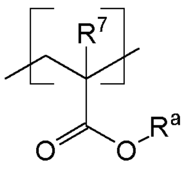
(2)

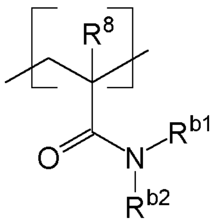

(3)

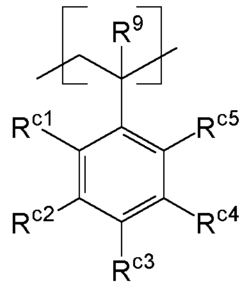

(4)

worin

$R^1$-$R^9$, $R^a$, $R^{b1}$ und $R^{b2}$ H oder eine organische Gruppe sind,
$R^{c1}$-$R^{c5}$ H, Halogen oder eine organische Gruppe sind,
$L^1$ eine Einfachbindung oder eine zweiwertige Verknüpfungsgruppe ist und
*n1* eine ganze Zahl von 3-10.000 ist.

**4.** Medizinisches Schmierelement gemäß Anspruch 3, wobei $R^a$ eine Gruppe der Formel (5) oder eine stickstoffhaltige organische Gruppe,

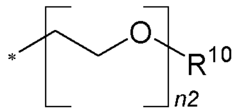

(5)

ist, worin n2 eine ganze Zahl von 1-10.000, $R^{10}$ H oder eine organische Gruppe ist und das Symbol "*" eine Bindungsstelle ist.

**5.** Medizinisches Schmierelement gemäß Anspruch 3 oder 4, wobei n1 135-10.000 ist.

**6.** Medizinisches Schmierelement gemäß irgendeinem der Ansprüche 1-5, wobei das Polymer der Schicht (b) ein vernetzter Körper mit einer vernetzten Struktur ist, die aus einer Vernetzungsmittelkomponente aus einer Struktureinheit der Formel (6) und/oder einer Verbindung der Formel (7) gebildet ist:

$-[CH_2-C(R^{11})(X)]-$ (6)

$Y-(R^{dm})_m$ (7)

worin

$R^{11}$ H oder eine organische Gruppe ist,
X, $R^{dm}$ jeweils unabhängig OH, Carboxy, Amino, Isocyanat, ein Oxazolinring, Epoxy, Vinyl, Ethinyl, Sulfanyl, Azid, Trialkoxysilyl oder eine Gruppe mit einer Säureanhydridstruktur sind,
Y eine *m*-wertige Verknüpfungsgruppe ist und
m eine ganze Zahl von $\geq 2$ ist.

**7.** Medizinisches Schmierelement gemäß Anspruch 6, wobei der Anteil der Vernetzungsmittelkomponente in dem vernetzten Körper der Schicht (b) 30-90 Masse-% beträgt.

**8.** Medizinisches Schmierelement gemäß irgendeinem der Ansprüche 1-7, wobei eine Oberfläche der Schicht (b) einer hydrophilen Behandlung unterzogen ist.

**9.** Medizinisches Schmierelement gemäß irgendeinem der Ansprüche 1-8, wobei das Basismaterial a aus einem Urethanharz, einem Siliciumharz, einem Fluorharz, einem Olefinharz und/oder einem Acrylharz gebildet ist.

**10.** Medizinisches Schmierelement gemäß irgendeinem der Ansprüche 1-9, wobei das Basismaterial a ein Siliciumharz

ist.

**11.** Verwendung des medizinischen Schmierelements gemäß irgendeinem der Ansprüche 1-10 als Element für eine medizinische Vorrichtung, ausgewählt aus einem medizinischen Schlauch, einem Führungsdraht, einem Endoskop, einer chirurgischen Nadel, einem chirurgischen Nahtmaterial, Forceps, einem künstlichen Blutgefäß, einem künstlichen Herz und einer Kontaktlinse.

**12.** Medizinische Vorrichtung, die das medizinische Schmierelement gemäß irgendeinem der Ansprüche 1-10 umfasst und aus einem medizinischen Schlauch, einem Führungsdraht, einem Endoskop, einer chirurgischen Nadel, einem chirurgischen Nahtmaterial, Forceps, einem künstlichen Blutgefäß, einem künstlichen Herz und einer Kontaktlinse ausgewählt ist.

## Revendications

**1.** Élément lubrifiant médical, comprenant un matériau stratifié comprenant

(i) un matériau de base (a) ;
(ii) disposée sur le matériau de base (a), une couche (b) contenant un polymère qui présente une structure de polysiloxane et contient

- un composant acide acrylique, un composant ester d'acide acrylique, un composant acrylamide, et/ou un composant styrène en tant que composant constitutif, et
- un groupe hydroxy, un groupe carboxy, un groupe amino, un groupe isocyanate, un cycle oxazoline, un groupe époxy, un groupe vinyle, un groupe éthynyle, un groupe sulfanyle, un groupe azide, un groupe trialcoxysilyle, et/ou une structure d'anhydride d'acide dans une molécule ; et

(iii) disposée sur la couche (b), une couche (c) contenant un polymère hydrophile sélectionné à partir d'une combinaison de deux ou plus d'une polyvinylpyrrolidone, d'un copolymère éther vinylique-anhydride de l'acide maléique, d'un polyéthylène glycol, d'un acide polyacrylique, d'un polyacrylamide et d'un acide hyaluronique.

**2.** Élément lubrifiant médical selon la revendication 1, dans lequel le polymère de la couche (b) est un polymère greffé présentant la structure de polysiloxane dans une chaîne greffée.

**3.** Élément lubrifiant médical selon la revendication 1 ou 2, dans lequel le polymère de la couche (b) contient une unité structurale de formule (1) et une unité structurale de formule (2), une unité structurale de formule (3), et/ou une unité structurale de formule (4) :

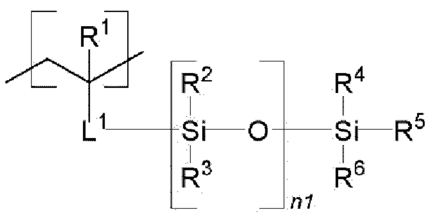
(1)

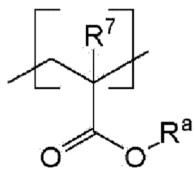
(2)

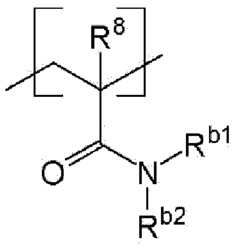
(3)

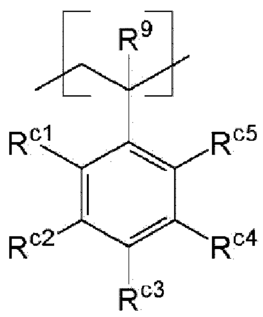
(4)

dans lesquelles

$R^1$-$R^9$, $R^a$, $R^{b1}$ et $R^{b2}$ sont H ou un groupe organique,
$R^{c1}$-$R^{c5}$ sont H, un halogène ou un groupe organique
$L^1$ est une liaison simple ou un groupe de liaison divalent, et
*n1* est un nombre entier de 3 à 10 000.

**4.** Élément lubrifiant médical selon la revendication 3, dans lequel $R^a$ est un groupe de formule (5) ou un groupe organique contenant un azote,

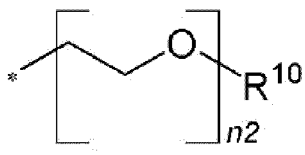

(5)

dans laquelle *n2* est un nombre entier de 1 à 10 000, $R^{10}$ est H ou un groupe organique, et le symbole « * » est un site de liaison.

**5.** Élément lubrifiant médical selon la revendication 3 ou 4, dans lequel n1 est de 135 à 10 000.

**6.** Élément lubrifiant médical selon l'une quelconque des revendications 1-5, dans lequel le polymère de la couche (b) est un corps réticulé présentant une structure réticulée formée d'un composant agent de réticulation d'une unité structurale de formule (6) et/ou d'un composé de formule (7) :

$-[CH_2-C(R^{11})(X)]-$ (6)

$Y-(R^{dm})_m$ (7)

dans lesquelles

$R^{11}$ est H ou un groupe organique,
X, $R^{dm}$ sont chacun indépendamment OH, un carboxy, un amino, un isocyanate, un cycle oxazoline, un époxy, un vinyle, un éthynyle, un sulfanyle, un azide, un trialcoxysilyle ou un groupe présentant une structure d'anhydride d'acide,
Y est un groupe de liaison *m*-valent, et
*m* est un nombre entier $\geq 2$.

**7.** Élément lubrifiant médical selon la revendication 6, dans lequel la proportion de composant agent de réticulation dans le corps réticulé de la couche (b) est de 30 à 90 % en masse.

**8.** Élément lubrifiant médical selon l'une quelconque des revendications 1-7, dans lequel une surface de la couche (b) est soumise à un traitement hydrophile.

**9.** Élément lubrifiant médical selon l'une quelconque des revendications 1-8, dans lequel le matériau de base a est formé par une résine uréthane, une résine de silicium, une résine fluorée, une résine oléfinique et/ou une résine acrylique.

**10.** Élément lubrifiant médical selon l'une quelconque des revendications 1-9, dans lequel le matériau de base est une résine silicium.

**11.** Utilisation de l'élément lubrifiant médical selon l'une quelconque des revendications 1-10 en tant qu'élément d'un dispositif médical sélectionné parmi une éprouvette médicale, un fil-guide, un endoscope, une aiguille chirurgicale, une suture chirurgicale, des forceps, un vaisseau sanguin artificiel, un cœur artificiel, et une lentille de contact.

**12.** Dispositif médical qui comprend l'élément lubrifiant médical selon l'une quelconque des revendications 1-10 et est sélectionné parmi une éprouvette médicale, un fil-guide, un endoscope, une aiguille chirurgicale, une suture chirurgicale, des forceps, un vaisseau sanguin artificiel, un cœur artificiel, et une lentille de contact.

FIG. 1

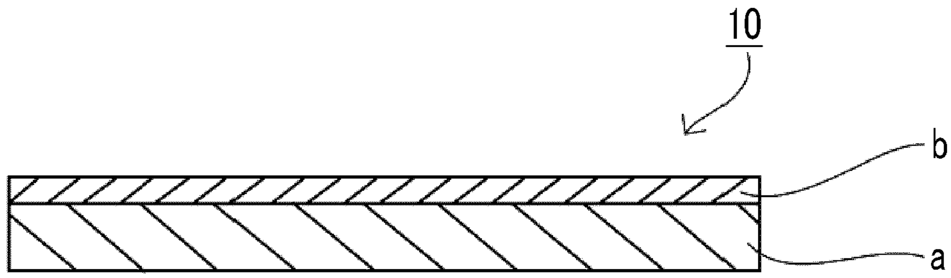


FIG. 2

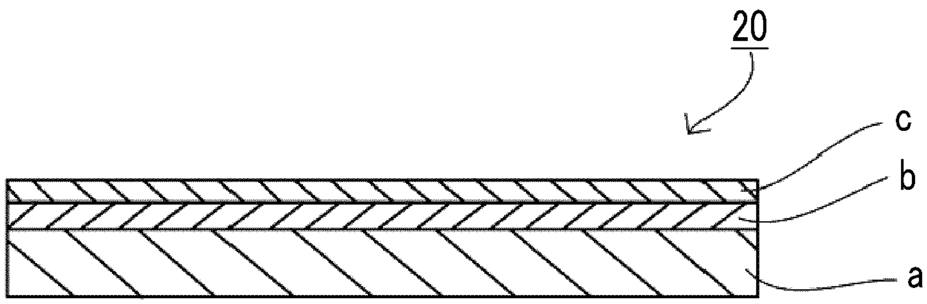

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2009261437 A